# EUROPEAN PATENT APPLICATION

(11) **EP 1 306 128 A1**
(43) Date of publication of application: **02.05.2003**
(21) Application number: 01204122.4
(22) Date of filing: 29.10.2001
(51) Int. Cl.: B01J 20/32, B01J 13/02, B01D 15/00, C12P 1/00

(54) **Sorptive composite materials**

(71) Applicant: Tenaxis Gmbh, 8093 Zürich (CH)
(72) Inventor: Held, Martin, 71332 Waiblingen (DE); Pellaux, René, 8004 Zürich (CH); Heile, Jens-Martin, 71640 Ludwigsburg (DE); Schenzle, Andreas Josef, 88690 Uhldingen-Mühlhofen (DE)
(74) Representative: Prins, Adrianus Willem

(57) **Abstract**

The invention relates to sorptive composite materials comprising a sorbent entrapped in a cross-linked acrylic polymer. These composites can be tailored for extraction and feeding of compounds from/to liquid phases or multiphase systems such as cell suspensions. Key features of the sorptive composite materials are stability (chemical, mechanical, biological and thermal), selectivity (physical and chemical), biocompatibility, and a characteristic upon which the sorptive composite materials can be separated from liquid or multi-phase systems. The invention also relates to techniques for the preparation of such sorptive composite materials as well as applications thereof.

## Description

### Field of invention

The present invention relates to methods for synthesis of sorptive composite materials that are suited for extraction, supply or separation of compounds. Specifically, the invention relates to the extraction of compounds from multiphase systems, wherein said composites are preferably biocompatible and comprise a cross-linked acrylic polymer entrapped sorbent. The invention relates further to the application of said sorptive composite materials for extraction of molecules from liquid or multi-phase systems, in particular cell suspensions.

### Background of the invention

Entrapment is a process wherein materials or compounds *e.g.* drugs, enzymes, organisms, or particles are enveloped in polymeric matrices that preferably consist of hydrogels.

Entrapment enables immobilization of both biological materials such as cells or enzymes as well as chemical agents and substances. The resulting entrapped materials are particularly useful for biotechnological or medical applications. Most abundant polymers for synthesis of entrapped materials are biological polymers such as carrageenan or alginate, though synthetic monomers such as acrylamide are also occasionally used.

Most publications on entrapment deal with immobilization of enzymes, microorganisms, plant or animal cells, or water-soluble compounds such as catalysts or pharmaceuticals. Up to now only a few examples for entrapment of particles into polymers have been reported. Today, particles are added to polymer solutions prior to solidification to facilitate production or processing of solutions or capsules or to increase stability of the capsules, rather than to synthesize new materials for novel applications.

In WO 00/36066 Neuser and coworkers (Neuser, Hsu *et al.* 2000), for instance, described the entrapment of finely distributed particles, especially silicates and carbonates, into carrageenan-hydrogels. The authors claim that adding particles to the carrageenan solutions facilitates processing and handling as solidification of the composites at lower temperatures is avoided.

Webbers and coworkers describe in US-Patent 5,916,789 (Webbers and Krijgsman 1999) the entrapment of enzymes and claimed that the stability of alginate or chitosan-hydrogels is increased if 1 - 3% activated carbon is added. Earlier Wang and coworkers (Wang and Nigam 1995) claimed in US 5,427,935 a method of more general character - whereby particles were referred to as anchor-materials.

However, Wang and coworkers (Nigam, Sakoda *et al.* 1988) described the entrapment of XAD-4 sorbent particles in agarose and alginate hydrogels and the application of these immobilized sorbents for protein recovery from unclarified broths and homogenates. The hydrogel acts as a protective non-fouling barrier that allows diffusion of the desired products, but it excludes colloidal solids such as whole cells, cell debris, and macromolecular precipitates.

Three principal methods or a combination thereof can be employed for synthesis of hydrogels for immobilization purposes.

*First,* ionotropic solidification, which refers to cross-linking of a water-soluble polyionic polymer (*e.g.* alginate, carrageenan, chitosan, *etc.)* with counter-ions (*e.g.* K⁺, Mg²⁺, Ca²⁺, Al³⁺, etc.) or charged polymers (*e.g.* poly(ethyleneimine), polylysine, polyphosphate, *etc.*). *Second,* thermal solidification, which refers to formation of hydrogels upon a temperature shift, usually cooling (*e.g.* agarose). *Third,* polymerization of functionalized monomers to give covalently cross-linked polymeric hydrogels (*e.g.* polyacrylamide).

The method of choice for synthesis of capsules or beads is ionotropic solidification in combination with a dripping technique for formation of droplets. A simple method is to use a syringe to drop the dissolved polymer into a hardening solution. Bead size however is limited by the syringe or cannula bore size and by the viscosity of the hydrogel-precursor solution. As a consequence, beads with a diameter less than 3 mm, and especially beads of less than 1 mm are difficult to produce. Naturally, internal and external mass transfer-rates through the hydrogel core are increased with decreasing diameter of the beads. Hence, small beads are found to be practicable for numerous applications.

Therefore, numerous other dripping techniques and devices, - some of which also allow scalable synthesis - have been developed.

In WO 96/28247 (Heinzen, Kuhn *et al.* 1996) and WO 99/44735 (Plüss-Wenziger, Widmer *et al.* 1999), for instance, devices for sterile entrapment that split up a laminar fluid jet by superimposition of an external vibration to give small droplets of uniform size are described. In EP 0167690 (Hommel, Sun *et al.* 1991) a technique for generation of droplets by aid of electromagnetic fields is claimed. DE 4424998 (Vorlop and Breford 1996) describes a method for droplet-formation that is based on physically 'cutting' liquid streams by aid of rotating wire-blades.

Syntheses of sorptive composite materials such as described in this patent are relatively independent of the method that is used for droplet-formation. In principle composite-synthesis can be done with all techniques, protocols or devices mentioned above as long as the particles that are subject to entrapment can pass through the nozzles.

Another important factor for hydrogel-bead synthesis is the rate at which droplets solidify after the precursors had been dripped into a hardening solution. As a rule, solidification has to take place at a rate that guarantees maintenance of a globular structure during and after gel formation. Hence, solidification has to take place very fast as otherwise the precursor solutions might be diluted or mixed with the hardening solution. In this context, ionotropic solidification has been found to be one of the most practical methods.

Alternatively hydrogels and the matter subject to entrapment can be polymerized *en bloc* and then mechanically broken down to particle size. However, this methodology often has failed to yield satisfactory results, as controlling of the particle size as well as manufacturing is difficult. Another technique is dispersion-polymerization. Hydrogel-precursors and the matter to be entrapped are dispersed in a second, non-miscible (typically organic) phase. Once droplets of desired size are formed the solidification process is initiated by addition of a starter. This technique is described in US 4,647,536 (Mosbach and Nilsson 1987) for *e.g.* entrapment of enzymes in agar, carrageenan, chitosan, polyacrylamide, gelatin, fibrinogen, agarose, or collagen hydrogel-particles. However, this method is limited by the physicochemical properties of both hydrogel-precursors and entrapped matter. Large differences in density or solubility in the second phase, for instance, may lead to a non-homogeneous dispersion and subsequently to a low quality of the hydrogel-beads.

As noted above biopolymers are frequently used as hydrogel-precursors. However, the applicability of these materials (alginate, carrageenan, etc.) under process conditions often suffers from the low chemical, mechanical, and thermal stability of such ionotropically formed hydrogels. One of the major obstacles for applicability is decay of the hydrogel-beads upon loss of counter-ions. Unfortunately, 'bleeding' of counter-ions occurs if hydrogels are used in aqueous solutions of other ionic strength than the hardening solution. Furthermore, culture media are usually rich in chelators such as phosphates, which effectively 'extract' two or more-fold charged metal ions such as used for cross-linking. In addition, media are often incubated in agitated compartments such as shaking-flasks or bioreactors. Under these conditions attrition and hydrogel-decay is accelerated.

Many biological hydrogel-precursors are de-polymerized upon heat-sterilization but the precursor solutions are prone to rotting or fouling. Special measures are thus required to handle biological hydrogel-precursors - especially on a larger scale.

For these reasons numerous materials essentially fail to serve the purpose they have been designed for, namely the application in biotechnology.

However, ionotropically solidified capsules can be stabilized upon the addition of another polymer that serves as a poly-counter-ion for the hydrogel-precursor. Lim and Sun (Lim and Sun 1980) published a method for entrapment of Langerhans' cells into alginate-hydrogels. The beads were post-synthetically treated with polylysine, which increased their stability. This method is still widely used, at least as far as entrapment of living cells is concerned. Analogously carrageenan hydrogels can be treated with cross-linking agents such as poly(ethyleneimine) (Chao, Haugen *et al.* 1986).

Other measures for increasing stability are based on the introduction of covalent cross-linking rather than on another ionotropic criterion. A recent example is the work of Soon-Shiong and coworkers published in US patents 5,700,848 (Soon-Shiong, Desai *et al.* 1997), 5,837,747 (Soon-Shiong, Desai *et al.* 1998) and 5,846,530 (Soon-Shiong, Desai *et al.* 1998). They described the synthesis of alginate-acrylesters that were later used as hydrogel-precursors. In a first step hydrogel-beads were formed upon ionotropic solidification of the functionalized alginate. In a second step, beads were covalently cross-linked by light-induced free radical polymerization of the acrylic functionality. This method yielded beads of superior stability but chemical synthesis of alginate-acrylester-precursors is laborious.

In contrast, the direct use of acrylic polymers, *e.g.* polyacrylamide, for entrapment is a comparatively simple and inexpensive method.

Watanabe and coworkers claim in US 4,421,855 (Watanabe, Sakashita *et al.* 1983) a method for entrapment of cells in polyacrylamide *en bloc.* Later Mosbach and coworkers claimed in US 4,647,536 (Mosbach and Nilsson 1987) a method for entrapment of biological materials into polyacrylamide by dispersion-polymerization. For reasons that have been discussed before both methods are of limited applicability for synthesis of sorptive composite materials such as introduced in accordance to the present invention.

Desirably, a process for the preparation of composite beads should comprise the following features. *First,* it should be possible to prepare beads by a dropping method and solidification should therefore be very fast. *Second,* beads should be chemically, mechanically, and thermally stable. *Third,* all reagents should be relatively inexpensive and the reaction has to be easily controllable, maintainable and scalable.

Methods for *in situ* product recovery (ISPR) - also often referred to as 'extractive biotransformations' - are of considerable interest for life science and biotechnological industries. Solid phases often have been found to be particularly useful for ISPR, downstream processing, and product polishing as they usually have a much higher selectivity than liquid extractants. The selectivity of solid phase sorbents can be attributed to numerous modes of molecular recognition or a combination thereof.

Other desirable features are good separability from turbid reaction liquids such as cell suspensions and regenerability as well as high stability and capacity. However, sorbents for ISPR in biotechnological processes should also be 'biocompatible', meaning they should essentially not cause any detrimental effects on the catalytically active elements, for instance, cells, microorganisms or enzymes. So far no satisfactory materials have been specifically designed for these purposes.

Nevertheless, some commercially available solid phase sorbents could in principle be suited for ISPR under certain conditions or in specific processes. Bulk sorbents such as hydrophobic polystyrene-based resins for instance have been frequently used for extraction of *e.g*. antibiotics or fine-chemicals from bacterial cultures (Held 2000).

Unfortunately, ion-exchangers or chromatography materials (aluminum oxide, titanium oxide, silica gel, etc.) are often not suited for ISPR as these sorbents may lack biocompatibility. Furthermore most of these materials are finely dispersed and are therefore not separable from biocatalytically active elements. For these reasons, regeneration and elution of these materials is complicated.

Wang and coworkers (Nigam, Sakoda *et al.* 1988) showed that the sorptive properties of sorbent materials can be maintained if they are entrapped in hydrogels formed from calcium alginate. As the sorbent is entrapped in a gel-shell of comparatively big diameter, sorbents can be forthwith recovered from multi-phase systems by for instance sieving. Furthermore, hydrogels are very often biocompatible, meaning that they do not mediate negative effects on cell viability or catalytic activity. The hydrogel materials disclosed by Wang and coworkers, however, have the disadvantage that their stability is insufficient for many practical purposes.

An example describing the use of a hydrogel-based material for ISPR from bacterial cultures is published in WO 00/73485 (Stark, Marison *et al.* 2000). The authors described the entrapment of organic liquid phases in alginates and a method for application of these materials for ISPR. More often, hydrogels have been used for extraction of inorganics. Pohl and coworkers (Pohl 1997) described in US 5,648,313 the application of biopolymeric sorbents for removal of heavy-metal ions from aqueous solutions. Similarly, Min and coworkers (Min and Hering 2001) employ Fe(III)-doped calcium alginate for sorption of arsenate or selenate.

### Summary of the invention

The present invention provides sorptive composite materials comprising a sorbent, preferably in the form of solid particles entrapped in a cross-linked acrylic polymer. The invention also provides a process for preparing these sorptive composite materials and their use in particular for ISPR in biotechnological multiphase systems. Claimed is also a method for the reversed effect of the application, namely for in situ supply of compounds.

A sorptive composite material according to the invention is highly suitable for ISPR as the cross-linked acrylic polymer used for entrapment typically has substantially no - or a negligible - effect on viability, growth characteristics or biocatalytic activity of biological elements such as cells, microorganisms, or enzymes. The cross-linked acrylic polymer envelops substantially most or all of the sorbent's surface. Therefore direct contact between biological elements and sorbents is either avoided or significantly reduced. However, the physicochemical properties of a sorptive composite material according to the invention can be tailored such that compounds subject to extraction still penetrate the cross-linked acrylic polymer. Once into contact with the sorbent these compounds are bound and easily recovered by elution after removal of the sorbent from the multiphase system by for instance sieving. The cross-linked acrylic polymer therefore functions as a biocompatible filter or buffer that is readily penetrated by some but not all of the ingredients of multiphase systems.

Thus, a sorptive composite material according to the invention has the distinct advantages of biocompatibility, separatability, selectivity (*e.g.* by adsorption), stability (*e.g.* essentially no disintegration under screening, culturing, or process conditions), and scalability. Furthermore, they can be prepared in a dripping method allowing their scalable synthesis at reasonable costs.

### Detailed description of the invention

The term sorbent as used herein refers to an inorganic or organic material that is capable to sorb a sorbate. Possible interactions that induce the sorbing include both adsorption and absorption. A sorbent to which a sorbate is sorbed may be referred to as a sorbent-sorbate complex.

Preferred sorbents include inorganic solids such as elementary metals, nonmetals and their compounds. A preferred metal is gold. Preferred compounds are oxides, hydroxides, carbonates, silicates, phosphates, sulfates, and halides. Preferred oxides are the oxides of aluminum, magnesium, silicon, titanium, zirconium. Further preferred are porous aluminum oxide and porous silica gel, *e.g.* as commercially available for chromatographical applications. Further preferred is modified silica, *e.g.* as commercially available for chromatography and solid phase extraction. Examples include octadecyl, octyl, cyclohexyl, phenyl, ethyl, cyanopropyl, aminopropyl and propanediol bonded porous silica. Further preferred silicates are fumed silica, diatomaceous earth, celite®, talcum, magnesium silicates as such but not limited to florisil®, zeolites as such but not limited to molecular sieves, clays as such but not limited to kaolin, mormorillonite, organically modified montmorillonites, bentonite, fullers earth. Further preferred are other inorganic solids with sorptive properties such as hydroxyapatite, graphite and activated carbon.

Preferred sorbents further include organic solids in the form of naturally occurring or chemically produced polymers. Preferred naturally occurring organic polymers are poly(hydroxyalkanoates), polylactate, polybutyrate and polysaccharides. Further preferred polysaccharides are cellulose and cellulose derivatives as well as agarose and agarose derivatives. Preferred chemically produced polymers are cross-linked polystyrenic resins, chemically functionalized cross-linked polystyrenic resins, cross-linked polyacrylic resins, and chemically functionalized cross-linked polyacrylic resins. Further preferred are chemically functionalized cross-linked polystyrenic and polyacrylic resins such as commercially available as weak or strong cation or anion exchangers. Further preferred chemically produced polymers are chelating resins, mixed bed resins, affinity resins and molecular imprinted polymers.

Cross-linked acrylic polymers in the context of the invention are copolymers synthesized from acrylic monomers and a cross-linking agent.

Preferred monomers are water-soluble acrylic acids, acrylates and acrylamides. Further preferred acrylic acids are acrylic acid and methacrylic acid and salts of acrylic acid and methacrylic acid. Further preferred acrylamides are acrylamide and N-(hydroxymethyl)acrylamide.

Preferred cross-linking agents are bifunctional acrylic monomers, preferably N,N'-methylenebisacrylamide and N,N'-ethylenebisacrylamide.

A sorptive composite material according to the invention preferably has the form of a hydrogel. This means that the material has taken up sufficient water to form a material having the properties of a gel. Typically, a hydrogel has the form of a water-swollen, rigid, three dimensional network of cross-linked, hydrophilic macromolecules. The water content in the gel may range between 20 and 95 wt.%.

A sorptive composite material according to the invention may further comprise an aqueous solute, preferably water, to which solvents, salts, buffers, surfactants, acids, or bases or other additives might have been added. Other additives can for instance be magnetic materials, density-influencing agents, ionotropic gelling agents, gelling-inducing agents, thickening agents and combinations thereof. Preferred are sorptive composite materials that contain water, whereby said water and the cross-linked acrylic polymer form a hydrogel.

Further preferred are sorptive composite materials in the form of particles having a diameter in the range of 0.2 - 4.0 mm or sorptive composite materials in the form of a coating having a thickness in the range of 0.1 - 50 mm.

Further preferred are sorptive composite materials preferably comprising 2 - 30 wt% acrylic monomer, 0.01 - 2 wt% cross-linking bifunctional acrylic monomer, 0.1 - 80 wt% sorbent and varying amounts of additives in aqueous solute, preferably water. Further preferred are sorptive composite materials preferably comprising 5 - 15 wt% acrylic monomer, 0.05 - 1 wt% cross-linking bifunctional acrylic monomer, 5 - 30 wt% sorbent and varying amounts of additives in water. The amount of additives incorporated is generally determined by the desired properties of the composite materials and by the synthetic procedure employed for their production.

An ionotropic gelling agent is a water-soluble ionic polymer, which may form a gel with the help of a gelling inducing agent. An ionotropic gelling agent such as preferably used in the context of the invention is selected from the group of ionic polysaccharides and other natural or synthetic ionic polymers. Preferred ionic synthetic polymers are poly(diallyldimethylammonium chloride), poly(ethyleneimine) and polylysine. Preferred polysaccharides are the water-soluble salts of alginate, pectate, chitosan, carrageenan as well as cellulose derivatives, especially carboxymethylcellulose and cellulose esters. A preferred alginate is aqueous sodium alginate, preferably in a concentration of 0.5 - 5 wt%, more preferably 1 - 3 wt%, based on the weight of the sorptive composite material. Further preferred is sodium alginate with a viscosity of about 250 cps for a 2 wt% solution at 25°C. Such sodium alginate is commercially available, for instance from Sigma Chemical Company, St. Louis, Mo, USA.

A gelling inducing agent is a mono- or polyvalent ion or ionic polymer, which induces gelation of an ionotropic gelling agent. Preferred gelling inducing agents for inducing the gelation of sodium alginate are positively charged bi- and trivalent water-soluble metal salts and positively charged water-soluble polymers such as chitosan or polylysine. A preferred hardening solution for induction of the gelation of sodium alginate contains water-soluble salts of bivalent calcium as gelling inducing agent, preferably in a concentration range of 0.01 - 2 M, more preferably in a concentration range of 0.05 - 0.2 M.

According to the invention, a thickening agent is a compound that increases the viscosity of a solution, preferably an aqueous solution, preferably mixtures of aqueous acrylic monomers and a cross-linking agent. Preferred thickening agents are poly(ethyleneglycol) and poly(vinylalcohol). Further preferred is a poly(ethyleneglycol) with an average molecular weight in the range of 5000 - 40000, used in a concentration in the range of 0.5 - 10 wt% in a reaction mixture comprising acrylic monomers and a cross-linking agent.

According to the invention, a magnetic compound is preferably a finely powdered compound that is attracted by a permanent magnet or an electromagnet. Magnetic compounds are preferably chosen from the group of compounds comprising metals, metal alloys and metal oxides. Further preferred metal oxides are ferrites, further preferred ferrite is magnetite, further preferred is magnetite with a particle size smaller than 5 microns.

According to the invention, density-influencing agents are finely powdered water-insoluble compounds upon addition of which as an additive the density of sorptive composite materials is influenced. Density-influencing agents are preferably elementary metals, nonmetals and their compounds, more preferred metals are precious metals.

A sorptive composite material according to the present invention is preferably prepared by free radical polymerization from a mixture containing acrylic monomer, cross-linking agent, and water. Additives such as magnetic compounds, density-influencing agents, thickeners, ionotropic gelling agents, ionotropic gelling inducers may be added. Further a commonly used polymerization initiator and accelerator such as potassium persulfate and N,N,N',N'-tetramethylethylenediamine may be added. Different methods for initiating and accelerating the polymerization such as the application of radiation, preferably ultraviolet light or X-ray, or heat may be used.

Preferably, the sorptive composite materials are synthesized as particles, preferably globular particles, preferably with a particle size of 0.2 to 4 mm. In order to synthesize globular particles of a sorptive composite material a mixture of acrylic monomer, cross-linking agent, sorbent, ionotropic gelling agent and water is prepared. Addition of polymerization accelerator may be desired to promote the reaction but is not a requirement. Preferably, the mixture is prepared by combining aqueous solutions of acrylic monomer, cross-linking agent and ionotropic gelling agent followed by addition of the polymerization accelerator. Preferably such a mixture comprises 2 - 30 wt% acrylic monomer, 0.01 - 2 wt% cross-linking bifunctional acrylic monomer, 0.5 - 5 wt% ionotropic gelling agent, 0.1 - 40 wt% sorbent, and 0.05 - 0.5 wt% polymerization accelerator in water. More preferred is a mixture of 5 - 15 wt% acrylic monomer, 0.05 - 1 wt% cross-linking bifunctional acrylic monomer, 1- 3 wt% ionotropic gelling agent, 5 - 30 wt% sorbent, 0.1 - 0.5 wt% polymerization accelerator in water.

By way of example, this mixture may be prepared by mixing 12 ml 4 wt% aqueous sodium alginate, 5 ml water, 8 ml 40 wt% aqueous acrylamide and 4 ml 2 wt% aqueous N,N'-methylenebisacrylamide, 8 g aluminum oxide and 1 ml 10 wt% aqueous N,N,N',N'-tetramethylethylenediamine to give a final composition of 8.40 wt% acrylic monomer, 0.21 wt% cross-linking agent, 1.26 wt% ionotropic gelling agent, 21.00 wt% sorbent, 0.26 wt% polymerization accelerator and 68.87 wt% water.

Droplets of the mixture are dripped into a hardening solution, which comprises an aqueous phase, preferably water, and an ionotropic gelling inducer. Of the mixture droplets might be prepared by various techniques. If the required size of droplets is in the range of 2 mm to 4 mm they may be prepared by pumping the mixture through an orifice of appropriate size. If smaller droplets are required, standard techniques for droplet-formation such as laminar jet break-up technology and commercially available equipment may be used. The hardening solution preferably contains a polymerization inducer such as potassium persulfate. Preferably the concentration of potassium persulfate in the hardening solution is in the range of 0.02 - 5 wt%, more preferably in the range of 0.5 - 2 wt%. Heat is preferably applied to the hardening solution to accelerate the polymerization process although this is not an essential requirement. The temperature for heat treatment is in the range of 20°C to 95°C, preferably in the range of 50°C to 70°C. Upon submersion of the droplets in the hardening solution beads are formed due to fast reaction of the ionotropic gelling agent with the ionotropic gelation inducer. The beads comprising acrylic monomer and cross-linking agent are then allowed to undergo free radical polymerization to form a cross-linked acrylic polymer. During this process the hardening solution might be stirred for keeping the beads in motion and avoiding their agglomeration. When the polymerization is completed the beads are separated from the hardening solution by sieving, for instance, and washed with water.

An ionotropic gel which may be present in the sorptive composite material may be destabilized and subsequently partially or totally removed by treatment with a chelating agent such as water-soluble salts of phosphate, citrate or ethylene diamine tetraacetate (EDTA). Preferably, removal of the calcium alginate gel is done by repeated treatment of the particles with an aqueous solution of sodium citrate or sodium phosphate, preferably in the concentration range of 0.01 - 0.2 M, more preferably in a concentration of 0.1 M. The resulting particles of the sorptive composite material may be dehydrated at temperatures in the range from 0 - 100°C. The dehydration may be effected at normal pressure or under reduced pressure. Preferred is the dehydration at a temperature of 50° and at a pressure of 10 - 20 mbar. Resolvatisation of the dehydrated sorptive composite material may be effected with help of hydrophilic solvents. Examples for hydrophilic solvents are water, acetonitrile, and alcohols such as methanol, ethanol or isopropanol. The resolvation may be effected under cooling, heating, or at room temperature. The resolvation may be effected at normal pressure or under elevated pressure. Preferred is resolvation in water or aqueous buffers at a temperature of 121°C in an autoclave.

According to the invention, an alternative procedure for the preparation of a sorptive composite material in the form of globular particles comprises the preparation of a mixture of acrylic monomers, cross-linking agent, magnetic compound, density-influencing agent, thickening agent, sorbent and water. Addition of polymerization accelerator may be desired to promote the reaction but is not a requirement. Preferably the mixture is prepared by combining aqueous solutions of acrylic monomer, cross-linking agent, thickening agent followed by addition of the polymerization accelerator. Preferably such a mixture comprises 2 - 30 wt% acrylic monomer, 0.01 - 2 wt% cross-linking bifunctional acrylic monomer, 0.5 - 10 wt% thickening agent, 0.1 - 40 wt% sorbent, and 0.05 - 0.5 wt% polymerization accelerator in water. Further preferred is a mixture of 5 - 15 wt% acrylic monomer, 0.05 - 1 wt% cross-linking bifunctional acrylic monomer, 2 - 10 wt% thickening agent, 5 - 30 wt% sorbent, and 0.1 - 0.5 wt% polymerization accelerator in water.

Droplets of the mixture are dripped into a hardening solution comprising a hydrophobic phase. Preferred hydrophobic phases are oils such as silicon oil or plant oils. Further preferred are silicon oils with densities in the range of 0.95 - 0.98 kg per liter and viscosities in the range of 300 - 1000 cps. Heat is applied to the hardening solution to promote the polymerization of the acrylic monomer. The temperature applied to the hardening solution is preferably in the range of 50°C to 95°C, more preferably in the range of 60°C to 90°C. The droplets are allowed to undergo free radical polymerization to form beads of cross-linked acrylic polymer and may be separated by sieving.

In a preferred embodiment, sorptive composite material is provided in the form of a coating on a surface, preferably of glass or plastic vessels. Preferred are coatings having a thickness in the range of 0.1 to 50 mm. In order to prepare a coating of a sorptive composite material according to the invention, a mixture of acrylic monomer, cross-linking agent, sorbent and water is prepared. Addition of a thickener sometimes may be required to stabilize the sorbent particle suspension but is not a requirement. Addition of a chemical polymerization accelerator, or acceleration of the polymerization by physical measures such as radiation, preferably ultraviolet light or X-ray may be desired to promote the reaction but is not a requirement. Preferably such a mixture comprises 2 - 30 wt% acrylic monomer, 0.01 - 2 wt% cross-linking bifunctional acrylic monomer, 0.5 - 10 wt% thickening agent, 0.1 - 40 wt% sorbent, 0.05 - 0.5 wt% polymerization accelerator in water. Further preferred is a mixture of 5 - 15 wt% acrylic monomer, 0.05 - 1 wt% cross-linking bifunctional acrylic monomer, 2 - 10 wt% thickening agent, 5 - 30 wt% sorbent, 0.1 - 0.5 wt% polymerization in water. Polymerization inducer is preferably added as aqueous solution and under vigorous stirring. The mixture is then applied to the surface to be coated and allowed to undergo free radical polymerization.

A sorptive composite material as described herein may find utility for specific removal of compounds from liquid, preferably aqueous, phases. A preferred embodiment concerns the recovery of metabolites or compounds produced or modified by cells or of compounds whose production is catalyzed by enzymes or homogeneous or heterogeneous chemical catalysts, preferably in situ. Recovery consists in the specific capture and separation of such metabolites or compounds from processing liquids, preferentially suspensions, emulsions or aqueous solutions, in particular culture media and reaction liquors, such as used in chemistry, cell biology, biotechnology, microbiology, or pharmacy.

The term cell as used herein is intended to encompass any virus, prokaryotic or eukaryotic cell, preferably microorganism, plant cells, or animal cells. Preferably, the cell is a bacterium, archaeum, alga, fungus, protozoan, plant cell, animal tumor cell, in particular mammalian or insect tumor cell, or hybridoma. The cell can also be a transiently transfected plant or animal cell, or a component of a mycelium, in particular a filamentous bacterium or filamentous fungus. Other possibilities include integral cellular components of a tissue, in particular of algal, fungal, plant or animal tissue, and cellular components of an intact symbiotic cohabit, in particular of a coral, sponge or lichen.

The term enzyme is intended to refer to any biomacromolecule wherein said biomacromolecules preferably have catalytic activity. Preferentially, the enzyme is a protein with catalytic activity that is derived from eu- or prokaryotic cells, preferably from naturally occurring wild type cells, B cell hybridomas, or recombinant cells, in particular transformed bacteria or fungi, or transiently or stably transfected animal or plant cells. An enzyme's catalytic activity may have been generated, modified, or enhanced by means of genetic engineering or immunization (*e.g.* catalytic antibodies also referred to as abzymes). It is preferred that the enzyme has been purified to a certain extent or that it is non-purified in the form of a cell extract or of homogenized tissue.

Processing liquids, preferentially suspensions, emulsions or aqueous solutions, in particular culture media such as commonly used in biotechnology frequently contain chelating agents, in particular phosphates. At a variance to polymers commonly used for entrapment such as alginate or carrageenan, which are prone to disintegration in the presence of chelating agents, the sorptive composite materials disclosed herein are characterized by chemical stability in said processing liquids. Furthermore, cultivation of cells requires a sterile environment, which in turn requires that both processing liquids and cultivation facilities are sterilized, preferably by physical methods such as heat treatment, preferably by autoclaving. The sorptive composite materials disclosed herein are characterized by thermal stability, preferably over a temperature range of 0°C to 121°C. Furthermore, cultivation of cells usually requires that they are incubated in agitated media. To this end, cultivation media are regularly incubated in test tubes, shaking flasks, or bioreactors. In conditions that are typical for biotechnological processes and experiments and at a variance to polymers commonly described for entrapment such as alginate or carrageenan, which upon agitation are prone to attrition and disintegration, the sorptive composite materials disclosed herein are characterized by great mechanical stability.

When applied in the form of free particles suspended in processing liquids, many of the sorbents used in the sorptive composite materials disclosed herein are toxic for living cells or inhibitory for enzymes. Moreover, heterogeneous chemical catalysts and free sorbents may interfere with each other. These effects are mainly contact-dependent. One advantage of entrapped sorbents as described herein is that cells, enzymes or heterogeneous chemical catalysts essentially do not come into direct contact with the entrapped sorbents but primarily only with the sorbent-entrapping cross-linked acrylic polymer, which on its own does not display toxic or inhibitory effects. Another reason for toxic or inhibitory effects of free, non-entrapped sorbents on living cells in aqueous solutions is that compounds that are essential for maintenance of certain properties of the cells, in particular catalytic activity, metabolic activity, survival or growth characteristics, are adsorbed and thus become unavailable for the cell. These compounds include media ingredients as well as cell-made molecules, specifically nutrients, antibiotics, vitamins, amino acids, proteins, hormones or growth factors. Similarly, enzyme stability or activity is often dependent on co-substrates or cofactors that might be withdrawn by free sorbents. Entrapment of sorbents into cross-linked acrylic polymers can establish a barrier allowing those compounds to predominantly remain freely available in the aqueous phase. Hence, entrapment of sorbents into cross-linked acrylic polymers can lead to prevention, delay or weakening of toxic or inhibitory effects of sorbent particles on cells or enzymes or delay or weakening of interferences between sorbent particles and heterogeneous chemical catalysts.

Another major drawback using free sorbents suspended in processing liquids for in situ product recovery lies in the technical difficulties encountered in further processing steps. These include the difficulty to separate the free sorbents from other components present in the processing liquid, namely cells or enzymes. A prominent consequence of this complication is clogging of sorbents, which impedes subsequent washing and elution steps thus leading to lower purity of eluates. Furthermore recycling of the adsorbent becomes impossible. Furthermore, a process designed for specific capture and recovery of more than one sorbate may include more than one sorbent. Free sorbents are hardly separable from each other and the different sorbates may therefore have to be eluted simultaneously, which defeats or eliminates the advantage of using sorbents with different specificity. The manageable size of the sorptive composite materials disclosed herein (>0.2 mm) confers to the entrapped sorbents the characteristic of separability, implemented by different modes of separation as for instance sieving. Different sorptive composite materials can be synthesized with different sizes (0.2 to 4.0 mm), allowing the separation of one from another by sieving. The auxiliary entrapment of magnetic compounds to the sorptive composite materials allows the separation by magnetic attraction, thus application of an additional separating principle. Similarly, entrapment of a compound with a certain density allows tailoring of the density, and therefore tailoring of its sedimentation rate or its upwelling properties, which in turn allows separation of the sorptive composite materials by appropriate methods such as sedimentation, flotation, or centrifugation.

The recovery of compounds, in particular metabolites produced by cells, in particular in a bioprocess, usually requires close observation of the compounds profiles and kinetics in the medium. Due to the specific regulation of metabolic pathways, compounds and in particular metabolites may tend to appear and disappear within a certain timeframe. Furthermore, many compounds are unstable under the conditions of an experiment or process and tend to undergo post-synthetic modifications. Other compounds, in turn, are volatile and tend to evaporate or to be stripped out from reaction compartments. The sorptive composite materials disclosed herein can have a stabilizing property, allowing the accumulation of compounds that would otherwise be further metabolized, the stabilization of compounds that would otherwise undergo post-synthetic modifications, or the retention of compounds that would otherwise evaporate or be stripped out from the reaction compartment.

Many compounds formed by cells are toxic or inhibitory for the cells that produced them and the biotechnological production of such compounds has to be carried out under conditions that ensure that a certain toxicity threshold is not exceeded. This can severely affect the profitability of the process. The sorptive composite materials disclosed herein can be used to in situ extract such toxic or inhibitory compounds. This allows improving the performance of such processes by increasing spatial yields.

Alternatively, the sorptive composite materials can be applied to remove undesired side-products, whether they are toxic or not, or to sorb and separate impurities, in order to facilitate further production steps. In the broader sense, a sorptive composite material according to the invention can be used for extraction or sorption of substances that are present in liquid phases in nature, preferably in aqueous phases, such as seas, rivers, springs, lakes or ponds, or substances that are present in communal or industrial waste-water or process-water streams.

Another characteristic of the sorptive composite materials disclosed herein is that the cross-linked acrylic polymer entrapping the sorbent can be tailored such that it contributes to the selectivity of the entrapped sorbent. This selectivity can be achieved through the pore size or through the physicochemical properties of the cross-linked acrylic polymer. The mode of cross-linkage by bifunctional cross-linking agents governs the porosity. Based on the porosity the time until compounds come into contact with the entrapped sorbent can be different for different compounds, preferably such that bigger molecules have a lower or infinite penetration rate through polyacrylic hydrogels than smaller molecules. Similarly, the physicochemical properties of the polyacrylic hydrogels, preferably their ionic character, polarity or chemical functionalization pattern, can be tailored such that penetration rates of compounds through the hydrogel are different, thereby achieving that the time until compounds come into contact with the entrapped sorbent are different for different compounds.

A sorptive composite material according to the invention can also be used for the slow release, supply or feed of a sorbate or a mixture of sorbates to a solution, preferentially to an aqueous solution such as a culture medium. The sorbate can be a nutrient, in particular a carbon or nitrogen-source, or a non-vital compound to be modified by the cells. Slow release is required if said nutrient or compound is toxic for the cell at elevated concentrations. The sorptive composite materials are particularly useful if such nutrients or compounds are well water-soluble and have a relatively high vapor pressure, which means that they cannot be fed via the gas phase. Furthermore, in this context the sorptive composite materials are particularly useful for screening or lab-scale experiments, as here a simple experimental approach is rather required than a sophisticated technology such as two-liquid phase systems or fed-regimes.

A sorptive composite material according to the invention has the advantage that bound compounds can be eluted with aqueous or organic solvents or mixtures thereof or with gases.

The invention will now be further illustrated by the following, non-restrictive examples.

### Examples

### 1. Synthesis of polyacrylamide / aluminum oxide composites (diameter > 2 mm)

Aqueous sodium alginate (12 ml; 4 wt%; low viscosity, Sigma), 5 ml water, 8 ml 40 wt% aqueous acrylamide (purum, Fluka) and 4 ml 2 wt% aqueous N,N'-methylenebisacrylamide (purum, Fluka) were filled in a 100 ml beaker. Aluminum oxide (8 g; pH neutral; for TLC, Fluka) was added and the mixture was homogenized by stirring. Then 1 ml 10 wt% aqueous N,N,N',N'-tetramethylethylenediamine (puriss., absolute, Fluka) were added, and the suspension was filled into a syringe and dropped (syringe pump; rate of 10 ml per minute) through a 1.2 mm cannula into a beaker filled with 80 ml of a stirred solution of 1 wt% ammonium peroxodisulfate (MicroSelect, Fluka) in 0.1 M aqueous calciumchloride (purum, Fluka) at 50°C.

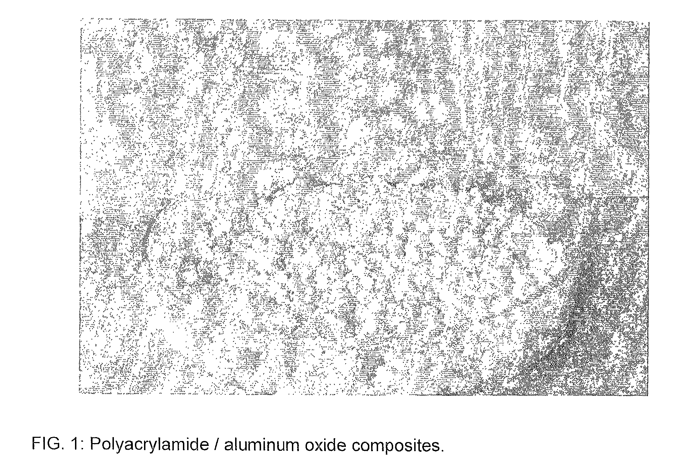

Beads of an average diameter of about 2.5 mm were formed upon submersion. After stirring for 30 min beads were recovered by sieving and washed with five aliquots of water (100 ml each). Then calcium alginate-polyacrylamide / aluminum oxide composites were immersed in 0.1 M aqueous tri-sodium-citrate-5,5-hydrate (extra pure, Merck) and allowed to equilibrate (6 h, RT). Beads were recovered by sieving and then washed (5 x 100 ml water). Citrate treatment and washing steps were repeated twice to remove the remaining calcium alginate, then the beads were stored in water. The resulting beads are shown in Figure 1.

50.00 g of the polyacrylamide / aluminum oxide composites were dried on air to a constant weight of 14.98 g. Rehydration in 500 ml of distilled water for 24 h gave 52.03 g polyacrylamide / aluminum oxide composites.

### 2. Synthesis of polyacrylamide / aluminum oxide composites (diameter < 2 mm)

A precursor-suspension according to procedure 1 was dripped into a hardening solution (droplet formation upon laminar-jet break-up technology, Nisco Engineering AG, Zurich). Production parameters were set on *A:* nozzle size: 0.5 mm, flow rate: 24 ml per minute, break-up frequency: 240 Hz for formation of 1.0 mm diameter beads, and on *B:* nozzle size: 0.3 mm, flow rate 10 ml per minute, break-up frequency: 210 Hz for formation of 0.6 mm diameter beads. Beads were post-synthetically treated and stored as described in procedure 1.

### 3. Synthesis of polyacrylamide / cetyl-montmorillonite composites

Synthesis was performed according to procedure 1, but with cetyl-montmorillonite (8 g, synthesized according to Huh, Song *et al.* 2000).

### 4. Synthesis of polyacrylamide / polystyrenic resin composites (subtype 1)

Synthesis was performed according to procedure 1 or 2, but with Amberchrome® CG-161M (8 g, Supelco, particle size 75 microns). The resin was filtered, washed with water and dried at air before use.

### 5. Synthesis of polyacrylamide / polystyrenic resin composites (subtype 2)

Synthesis was performed according to procedure 1 or 2, but with Amberlite® XAD-1180 (8 g, Supelco). The resin was filtered, washed with water and dried at air, crushed and sieved through a 0.2 mm sieve before use.

### 6. Synthesis of magnetic polyacrylamide / polystyrenic resin composites

Synthesis was performed according to procedure 4, but with Amberchrome® CG-161M (6 g, Supelco, particle size 75 microns) and magnetite (6 g, Aldrich, particle size < 5 microns).

### 7. Synthesis of polyacrylacid / polystyrenic resin composites

Polyacrylamide / polystyrenic resin composites (50 g, procedure 4) were covered by 200 ml 1 M aqueous sodium hydroxide and stirred for 24 hours. The composites were recovered by sieving and then washed (5 x 100 ml water).

### 8. Synthesis of poly(N-(hydroxymethyl)acrylamide)/polystyrenic resin composites

Synthesis was performed according to procedure 4, but with aqueous N-(hydroxymethyl)acrylamide (purum, Fluka).

### 9. Synthesis of (polyacrylamide / aminopropyl silica composite)-coating of a shaking flask

8 ml 40 wt% aqueous acrylamide (purum, Fluka) and 4 ml 2 wt% aqueous N,N'-methylenebisacrylamide (purum, Fluka) and 18 ml of water were stirred in a 500 ml shaking flask. Aminopropyl silica gel (8 g; for TLC, Merck LiChroprep®) was added and the mixture was homogenized by stirring. Then 1 ml 10 wt% aqueous N,N,N',N'-tetramethylethylenediamine (puriss., absolute, Fluka) were added. Finally 1 ml aqueous solution of 1 wt% ammonium peroxodisulfate (MicroSelect, Fluka) was added under stirring and the shaking flask is transferred to a water bath at 60° to allow the polymerization process to proceed. After 30 minutes reaction time the coating was washed (3 x 300 ml water) and stored in water (100 ml).

### 10. Synthesis of calcium alginate / aluminum oxide composites reference material used in the sorption experiments

Aqueous sodium alginate (100 ml; 2 wt%; low viscosity, Sigma) was homogenized with 21 g aluminum oxide (pH neutral; for TLC, Fluka). The suspension was filled into a syringe with a 1.2 mm cannula and dropped (syringe pump; 10 ml/min) into a beaker filled with 300 ml of a stirred hardening solution (0.1 M aqueous calcium chloride). Composite beads (average diameter 2.5 mm) were formed instantaneously upon submersion. After stirring for 30 min beads were removed by sieving, washed with five aliquots (each 100 ml) water, and stored in 0.1 M aqueous calcium chloride.

### 11. Sorption of 2-phenylphenol and 3-phenylcatechol on polyacrylamide / cetyl-montmorillonite composites

Polyacrylamide / cetyl-montmorillonite composites (10 g, procedure 3) were added to 100 ml of a stirred aqueous solution containing 100 mg/L 2-phenylphenol and 3-phenylcatechol, respectively. Samples were taken in regular intervals, acidified with 0.1 N hydrochloric acid and the amount of non-adsorbed 2-phenylphenol and 3-phenylcatechol was determined by highpressure liquid chromatography (HPLC).

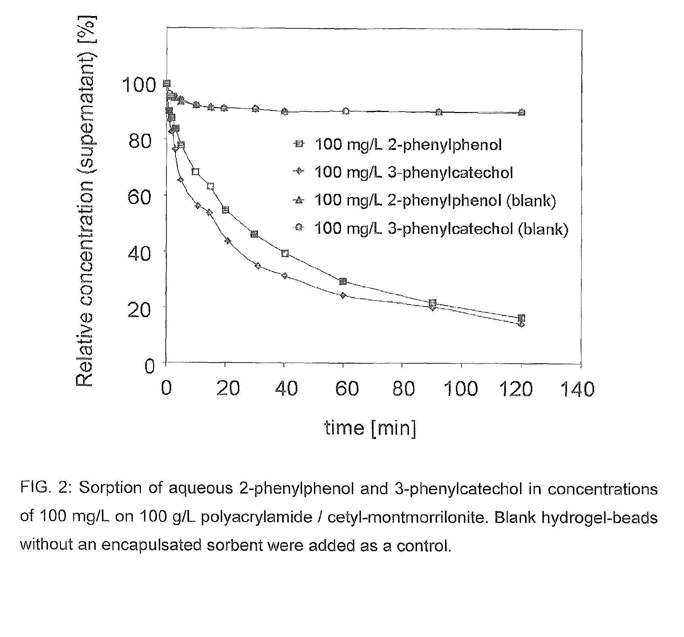

Figure 2 shows the 2-phenylphenol and 3-phenylcatechol concentrations in the supernatant as a function of time. The rate at which the supernatant is cleared by the polyacrylamide / cetyl-montmorillonite is comparable for both compounds.

### 12. Selective sorption of 3-phenylcatechol on polyacrylamide/ aluminum oxide composites

Polyacrylamide / aluminum oxide composites (10 g; procedure 1) were added to 100 ml of a stirred aqueous solution of 50 mg/L 2-phenylphenol and 50 mg/L 3-phenylcatechol, respectively. Aliquots were taken in regular intervals, acidified with 0.1 N hydrochloric acid, and concentrations were determined by HPLC.

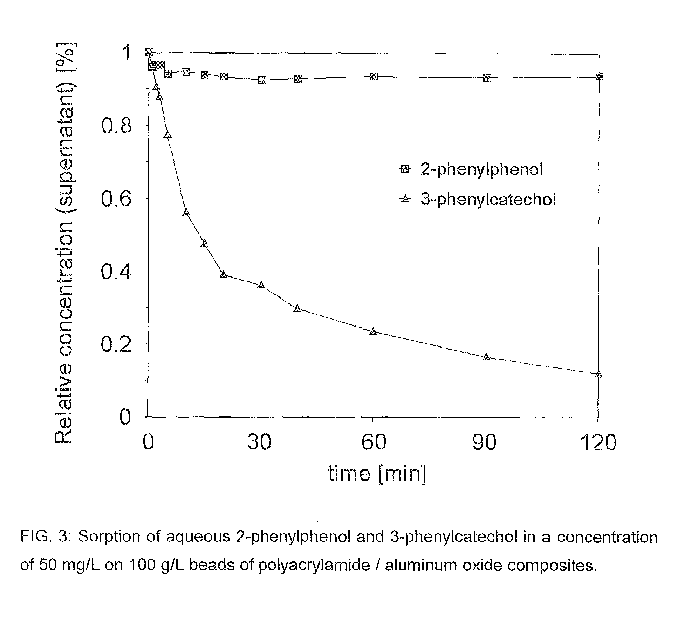

Polyacrylamide / aluminum oxide composites adsorb 2-phenylphenol and 3-phenylcatechol differentially (Figure 3). Approximately 88 % of all 3-phenylcatechol added was adsorbed within 120 minutes. However, within the same time only 7 % of all 2-phenylphenol added was adsorbed. Thus, polyacrylamide / aluminum oxide composites highly selectively adsorb 3-phenylcatechol but not 2-phenylphenol. The result is consistent with the property of aluminum oxide to selectively bind catechols but not phenols (Held 2000). Entrapment of aluminum oxide in polyacrylamide did not effect this property.

### 13. Sorption of riboflavin and proteins on polyacrylamide) /XAD®-1180 composites

Polyacrylamide / Amberlite® XAD-1180 composites (10 g, procedure 5) were added to 100 ml of a stirred aqueous solution containing 100 mg/L riboflavin (Sigma, Mr = 376.4 D) and hemoglobin (Fluka, from bovine blood, Mr = 64500 D), respectively. Samples were taken in regular intervals. Concentrations of non-adsorbed riboflavin and protein (Bradford) were measured spectrophotometrically.

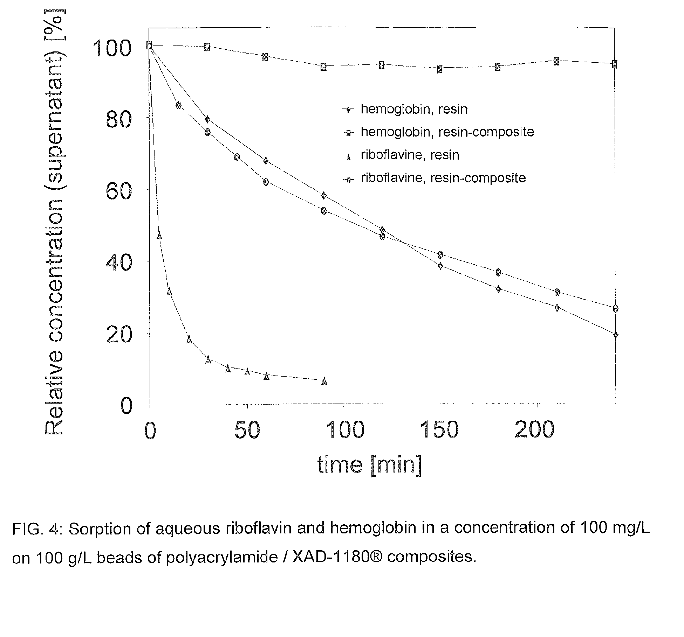

Figure 4 shows the riboflavin and hemoglobin concentrations in the supernatant as a function of time. Hemoglobin as well as riboflavin adsorbed well onto the free resin. Nevertheless, hemoglobin was not adsorbed by the composites, while riboflavin was, which indicates that the hydrogel strongly discriminates between hemoglobin and riboflavin.

### 14. Stability of polyacrylamide / aluminum oxide composites vs. calcium alginate / aluminum oxide composites towards phosphates and thermal treatment

10 g of polyacrylamide / aluminum oxide composites (procedure 1) or 10 g of calcium alginate / aluminum oxide composites (procedure 10) were each added to two 250 ml round shaking flasks containing 80 ml of either water or 50 mM phosphate buffer pH 7.0. One set of flasks was autoclaved whereas the other was left at room temperature. All flasks were then left overnight at room temperature. Polyacrylamide / aluminum oxide remained virtually unchanged, independently of thermal treatment and phosphate concentration. In contrast, calcium alginate / aluminum oxide beads incubated in 50 mM phosphate were partially disintegrated and considerable particle release was observed for both autoclaved and non-autoclaved calcium alginate / aluminum composites. Beads were reduced in size, blurred, swollen and partially solubilized. The viscosity of the liquor was increased, likely due to partial resolvation of highly viscous alginate precursors from the composite.

### 15. Stability of polyacrylamide / aluminum oxide composites and calcium alginate / aluminum composites in media containing phosphates as a nutrient

A typical phosphate-requirement for growth of microorganism on 1 wt% of a commonly used carbon-source such as glucose is approximately 4 mM. However, in batch cultures phosphate is often fed in excess (concentrations of up to 50 mM) because it is also added as a buffer component. The stability of the composites towards phosphate in these concentration ranges was assessed in shaking flask experiments. Therefore, calcium alginate / aluminum oxide composites were treated as described above but now in 4 mM phosphate. After storage over-night, calcium alginate / aluminum oxide composites showed little attrition as indicated by turbidity of the liquor after shaking the flasks by hand. Calcium alginate / aluminum oxide composites were then incubated on a rotary shaker (30°C, 130 rpm, amplitude 20 mm). Composite decay was regularly judged by eye. Under these conditions calcium alginate / aluminum oxide composites decay rapidly, independently on thermal pretreatment by *e.g.* autoclaving. After 90 hours of shaking, virtually all beads were disintegrated.

Polyacrylamide / aluminum oxide composites were added to demineralized water, to 4 mM phosphate and to 50 mM phosphate, and analogously treated. As indicated by the turbidity of the liquor (by eye) after shaking of the flasks by hand, polyacrylamide / aluminum oxide composites were stable over 150 h. Only a negligible amount of particles were released and the composite-beads retained their globular structure. However, as a side effect polyacrylamide / aluminum oxide and composites increased in diameter - at least by a factor of 2 - when incubated in water.

### 16. Biocompatibility of free aluminum oxide versus polyacrylamide / aluminum oxide composites: growth of Spingomonas sp. HXN-200

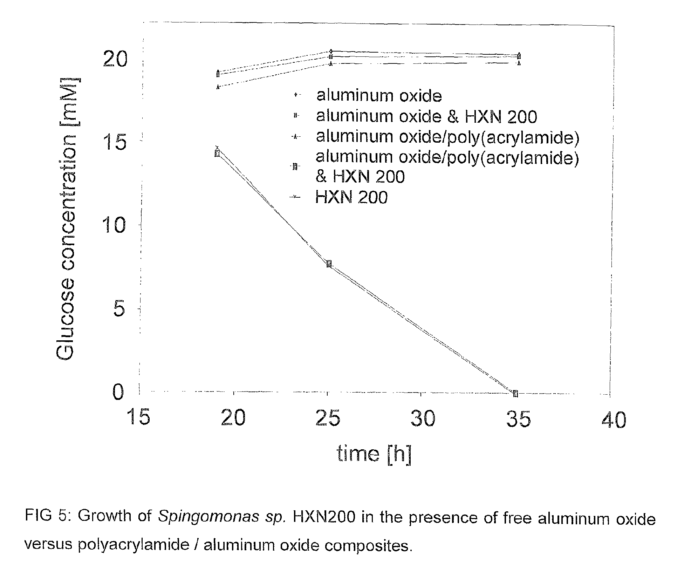

Biocompatibility of aluminum oxide and polyacrylamide / aluminum oxide composites was assessed in growth experiments. Therefore, 5 g of polyacrylamide / aluminum oxide composites, containing approximately 1 g of aluminum oxide, and 1 g of free aluminum oxide as a control, were autoclaved in 50 mM phosphate buffer pH 7.1. A carbon source (20 mM glucose) and salts were added as indicated (Held 2000) and flasks were inoculated 1:100 with an overnight culture of *Spingomonas sp.* HXN-200. As a reference, *Spingomonas sp.* HXN-200 was grown without sorbent material. As a control for glucose-adsorption, the composite was incubated in the medium without cells. Growth was indirectly assessed by measuring the glucose-concentration as a function of time (Trinder assay, Sigma Diagnostics). Figure 5 shows glucose consumption as a function of time. Free aluminum oxide completely inhibited growth of *Spingomonas sp.* HXN-200 whereas cell growth was regular in the presence of the corresponding amount of aluminum oxide if entrapped in polyacrylamide / aluminum oxide composites.

### 17. Biocompatibility of various polyacrylamide / sorbent composites with various microorganisms

Four bacterial strains were incubated in liquid culture and in the presence of polyacrylamide / sorbent composites. The microorganisms include two Gram-negative strains, *Spingomonas sp.* HXN-200 (Chang, Witholt *et al.* 2000) and recombinant *Escherichia coli* W3110(pGEc47) (Nieboer, Kingma *et al.* 1993), as well as two Gram-positive strains, *Rhodococcus rhodochrous* DSM 11097 and filamentous *Streptomyces arenae* DSM 40737 (Brünker, McKinney *et al.* 1999). The microorganisms were incubated in an appropriate growth media with and without various amounts of polyacrylamide / sorbent composites. Biocompatibility was assessed by either measuring optical density or glucose consumption or both. In addition, samples were regularly checked (light microscopy) for shape, size, and location of the cells. The results are summarized in table 1.

**Table 1:**

| Biocompatibility of polyacrylamide-entrapped sorbents with microorganisms. | | | | |
|---|---|---|---|---|
| Sorbent / Microorganism | *Escherichia coli* W3110 (pGEc47) | *Spingomonas sp.* HXN-200 | *Rhodococcus rhodochrous* DSM 11097 | *Streptomyces arenae* DSM 40737 |
| Aluminum oxide | biocompatible | Biocompatible | n.t. | n.t. |
| Amberchrom CG-71 M® | not tested (n.t.) | Biocompatible | n.t. | n.t. |
| Amberlite XAD-4® | n.t. | Biocompatible | biocompatible | n.t. |
| Amberlite XAD-7® | n.t. | Biocompatible | biocompatible | n.t. |
| Dowex 50W® | n.t. | Biocompatible | n.t. | n.t. |
| Dowex 50WX8400® | n.t. | n.t. | biocompatible | biocompatible |
| LiCliroprep® NH2 | n.t. | n.t. | biocompatible | biocompatible |
| LiChroprep® RP-2 | n.t. | n.t. | biocompatible | n.t. |
| Molecular sieve UOP | n.t. | Biocompatible | biocompatible | n.t. |
| type 13 | | | | |
| Montmorillonite K10 | n.t. | n.t. | biocompatible | n.t. |

### 18. In situ extraction of metabolites formed from berberine by biocatalyst TNXS-BBV5

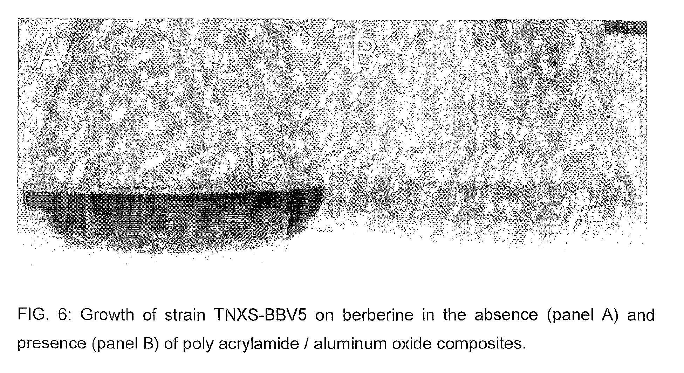

Growth of TNXS-BBV5 on berberine in liquid media (shaking flasks) is characterized by a lag-phase of 9 days. As growth proceeds the media is readily discolored brownish (Figure 6A), probably due to formation of humic acid-like structures as a result of metabolite-accumulation and polymerization (unpublished results).

However, in the presence of 12 g of polyacrylamide / aluminum oxide composites (80 ml minimal medium with 27 mg berberine) the lag-phase was significantly shortened to 1-2 days. Furthermore, no brown-color formation was observed (Figure 6B).

After depletion of berberine the composites were separated from culture broth and cells by filtration, washed with tap water, and eluted with 0.5 N hydrochloric acid. The eluate was analyzed by mass spectroscopy after chromatographic separation (HPLC-MS, Hewlett Packard Series 1100 HPLC-MS; stationary phase: Prontosil ODSH 120-C18H, 125x4.6 mm, 5 µl particles; mobile phase: acetic acid pH 6 and methanol gradient from 25 % methanol/75 % acetic acid to 100 % methanol in 6 min, flow rate 1.0 ml/min). In the eluate two metabolites (table 2) but no berberine was detected. The suggested structures of the metabolites indicate that both metabolites are likely to have catechol-functionalities.

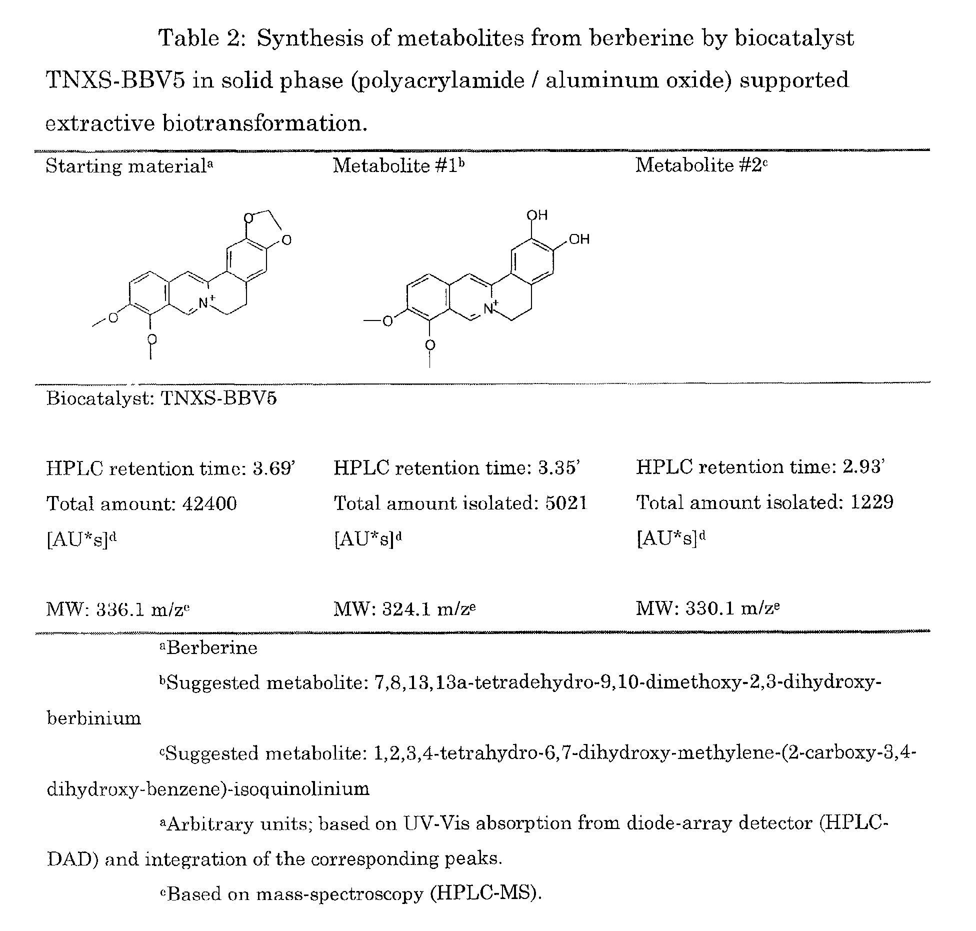

The results indicate that humification could be avoided upon instantaneous adsorption of metabolites by the composite material thus providing an elegant method for recovery and stabilization of these compounds. Furthermore, growth-inhibitory effects likely to be caused by metabolites or by their polymerization products were minimized.

### References

Brünker, P., K. McKinney, *et al.* (1999). "Isolation and characterization of the naphthocyclinone gene cluster from *Streptomyces arenae* DSM 40737 and heterologous expression of the polyketide synthase genes." Gene **227**: 125-135.

Chang, D., B. Witholt, *et al.* (2000). "Preparation of (S)-N-substituted 4-hydroxy-pyrrolidin-2-ones by regio- and stereoselective hydroxylation with *Spingomonas sp.* HXN-200." Organic Letters **2**: 3949-3952.

Chao, K. C., M. M. Haugen, *et al.* (1986). "Stabilization of *kappa*-Carragenan gel with Polymeric Amines: Use of Immobilized Cell as Biocatalysts at Elevated Temperatures." Biotechnology and Bioengineering **28**: 1289-1293.

Heinzen, C., O. Kuhn, *et al.* (1996). Encapsulation Equipment. WO 96/28247.

Held, M. (2000). Biocatalytic synthesis of 3-substituted catechols. Institute of Biotechnology. Zurich, Swiss Federal Institute of Technology: 119-142.

Hommel, M., A. M.-F. Sun, *et al.* (1991). Droplet generation. EP 01/67690.

Huh, J., D. Song, *et al.* (2000). "Sorption of Phenol and Alkylphenols from Aqueous Solution onto Organically Modified Montmorillonite and Applications of Dual-Mode Sorption Model." Separation Science and Technology **35**(2): 243.

Lim, F. and A. Sun "Microencapsulated islets as bioartificial endocrine pancreas" (1980). Science **210**: 908-910.

Min, J. H. and J. Hering, G. (2001). Iron(III)-Doped Calcium Alginate Gel Sorbents for Sorption of Arsenate and Selenate. US 6,203,709.

Mosbach, K. and K. Nilsson (1987). Method of encapsulating biomaterial in bead polymers. US 4,647,536.

Neuser, C., F.-L. Hsu, *et al.* (2000). Polymer-Containing Particle and Process for the Preparation thereof. WO 00/36066.

Nieboer, M., J. Kingma, *et al.* (1993). "The alkane oxidation system of *Pseudomonas oleovorans:* induction of the *alk* genes in *Escherichia coli* W3110(pGEc47) affects membrane biogenesis and results in overexpression of alkane hydroxylase in a distinct cytoplasmic membrane subfraction." Molecular Microbiology **8**: 1039-1351.

Nigam S. C., Sakoda A. and Wang H. Y. (1988). Bioproduct recovery from unclarified broths and homogenates using immobilized adsorbents. Biotechnology progress 4, 166.

Plüss-Wenziger, R., F. Widmer, *et al.* (1999). Method and device for capsulating microbial, plant and animal cells or biological and chemical substances. WO 99/44735.

Pohl, P. (1997). Method for Production of Adsorption Materials. US 5,648,313.

Soon-Shiong, P., N. P. Desai, *et al.* (1997). Gel Compositions prepared from Cross-linkable Polysaccharides, Polycations and/or Lipids and Uses therefor. US 5,700,848.

Soon-Shiong, P., N. P. Desai, *et al.* (1998). Cross-linkable Polysaccharides, Polycations and/or Lipids Useful for Encapsulation and Drug Release. US 5,837,747.

Soon-Shiong, P., N. P. Desai, *et al.* (1998). Macrocapsules prepared from Cross-linkable Polysaccharides, Polycations and/or Lipids and Uses therefor. US 5,846,530.

Stark, D., I. Marison, *et al.* (2000). Method and Device for In-Situ Extraction and Supply. WO 00/73485.

Vorlop, K.-D. and J. Breford (1996). Verfahren zur Herstellung von Teilchen aus einem flüssigen Medium. DE 4,424,998.

Wang, H. Y. and S. C. Nigam (1995). Hybrid Membrane Bead and Process for Encapsulting Materials in Semi-Permeable Hybrid Membranes. US 5,427,935.

Watanabe, I., K. Sakashita, *et al.* (1983). Production of acrylamide using immobilized cells. US 4,421,855.

Webbers, J. J. P. and J. Krijgsman (1999). Immobilized Enzyme. US 5,916,789.

## Claims

1. Sorptive composite material comprising a sorbent entrapped in a cross-linked acrylic polymer.

2. Sorptive composite material according to claim 1, wherein the sorbent is an inorganic solid chosen from the group elementary metals, nonmetals and their compounds.

3. Sorptive composite material according to claim 1, wherein the sorbent is an organic solid chosen from the group of natural and synthetic polymers.

4. Sorptive composite material according to any of the preceding claims, further comprising a sorbate sorbed to the sorbent.

5. Sorptive composite material according to any of the preceding claims, wherein the acrylic polymer is in the form of a hydrogel.

6. Sorptive composite material according to any of the preceding claims, comprising of from 0.1 - 80 wt% of sorbent, based on the weight of the sorptive composite material.

7. Sorptive composite material according to any of the preceding claims, further comprising an additive chosen from the group of ionotropic gelling agents, thickening agents, magnetic compounds and density-influencing agents, and combinations thereof.

8. Sorptive composite material according to claim 7, wherein the magnetic compound is chosen from the group of metals, metal alloys, metal oxides, and combinations thereof.

9. Sorptive composite material according to claim 7, wherein the density-influencing agent is chosen from the group of elementary metals, nonmetals and their compounds.

10. Sorptive composite material according to claim 7, wherein the thickening agent is chosen from the group of poly(ethylenglycols), poly(vinylalcohols), and combinations thereof.

11. Sorptive composite material according to claim 7, wherein the ionotropic gelling agent is an ionic polymer, preferably an ionic polysaccharide.

12. Sorptive composite material according to any of the preceding claims, having a particulate form with a diameter of from 0.2 to 4.0 mm.

13. Sorptive composite material according to any of the claims in the form of a coating having a thickness in the range of from 0.1 to 50 mm.

14. Process for preparing a sorptive composite material according to any of the preceding claims, wherein an acrylic monomer is polymerized in the presence of the sorbent and a cross-linking agent.

15. Process according to claim 14, comprising the steps of
- preparing a mixture of acrylic monomer, cross-linking agent, sorbent, an ionotropic gelling agent, a polymerization accelerator and water;
- dripping droplets of the mixture into a hardening solution comprising water and a gelling inducing agent upon which beads are formed;
- allowing the acrylic monomer to polymerize to form the acrylic polymer; and
- separating the beads from the hardening solution by sieving.

16. Process according to claim 14 or 15, wherein the acrylic monomer is chosen from the group of water-soluble acrylic acids, acrylates and acrylamides

17. Process according to any of the claims 14-16, wherein cross-linking agent is a bifunctional acrylic monomer.

18. Process according to any of the claims 15-17, wherein the ionotropic gelling agent is a water-soluble ionic polymer.

19. Process according to any of the claims 15-18, wherein the mixture comprises 2 - 30 wt% acrylic monomer, 0.01 - 2 wt% cross-linking agent, 0.5 - 5 wt% ionotropic gelling agent, 0.1 - 40 wt% sorbent, 0.05 - 0.5 wt% polymerization accelerator, and the balance being water.

20. Process according to claim 19, wherein the mixture comprises 5 - 15 wt% acrylic monomer, 0.05 - 1 wt% cross-linking bifunctional agent, 1- 3 wt% ionotropic gelling agent, 5 - 30 wt% sorbent, 0.1 - 0.5 wt% polymerization accelerator, and the balance being water.

21. Process according to any of the claims 15-20, wherein the hardening solution comprising water, a gelling inducing agent and an initiator.

22. Process according to claim 21, wherein the gelling inducing agent is a mono- or polyvalent ion or ionic polymer.

23. Process according to any of the claims 15-22 further comprising the step of removing gel formed by the ionotropic gelling agent by contacting the sorptive composite material with a chelating agent.

24. Process according to claim 23, wherein the chelating agent is a water-soluble salt of phosphate, citrate or ethylene diamine tetraacetate.

25. Process according to claim 14, comprising the steps of:
- preparing a mixture of acrylic monomer, cross-linking agent, sorbent, and a thickener in water;
- dripping droplets of the mixture into a hydrophobic phase;
- allowing the acrylic monomer to polymerize to form the acrylic polymer;
- allowing the acrylic polymer to cross-link upon which beads are formed; and
- separating the beads from the hydrophobic phase.

26. Process according to claim 25, wherein the thickening agent is a poly(ethyleneglycol) or a poly(vinylalcohol).

27. Process according to claim 25 or 26, wherein the hydrophobic phase is an oil.

28. Process according to any of the claims 25-27, wherein the mixture comprises 2 - 30 wt% acrylic monomer, 0.01 - 2 wt% cross-linking agent, 0.5 - 10 wt% thickening agent, 0.1 - 40 wt% sorbent, 0.05 - 0.5 wt% polymerization accelerator, and the balance being water.

29. Process according to claim 28, wherein the mixture comprises 5 - 15 wt% acrylic monomer, 0.05 - 1 wt% cross-linking agent, 2 - 10 wt% thickening agent, 5 - 30 wt% sorbent, 0.1 - 0.5 wt% polymerization accelerator, and the balance being water.

30. Process according to claim 14, wherein a mixture of the acrylic monomer, the cross-linking agent and the sorbent is formed, which mixture is applied to the surface of a substrate and the acrylic monomer is allowed to polymerize to form a acrylic polymer which is allowed to cross-link to form a coating of the sorptive composite material.

31. Process according to claim 30, wherein the mixture comprises 2 - 30 wt% acrylic monomer, 0.01 - 2 wt% cross-linking agent, 0.5 - 10 wt% thickening agent, 0.1 - 40 wt% sorbent, 0.05 - 0.5 wt% polymerization accelerator, and the balance being water.

32. Process according to claim 31, wherein the mixture comprises 5 - 15 wt% acrylic monomer, 0.05 - 1 wt% cross-linking agent, 2 - 10 wt% thickening agent, 5 - 30 wt% sorbent, 0.1 - 0.5 wt% polymerization accelerator, and the balance being water.

33. Process for sorbing a sorbate by bringing a sorptive composite material according to any of the claims 1-13 into contact with the sorbate.

34. Process according to claim 33, wherein the sorbate is a compound produced by a cell.

35. Process according to claim 34, wherein said compound is produced by culturing the cell in the presence of the sorptive composite material.

36. Process according to claim 34 or 35, wherein the cell is a virus, a prokaryotic or an eukaryotic cell.

37. Process according to claim 33, wherein the sorbate is a compound, which is synthesized using an enzyme, a heterogeneous or homogeneous catalyst.

38. Process according to claim 37, wherein synthesis of the compound is carried out in the presence of the sorptive composite material.

39. Process according to claim 33, wherein the sorbate is selectively sorbed from a mixture comprising other potential sorbates and wherein the sorptive composite material has a pore size which allows selective sorption of the desired sorbate.

40. Process according to any of the claims 33-39, further comprising the step of separating the sorbate sorbed to the sorptive composite material from other components in a cell culture or reaction mixture.

41. Process according to any of the claims 33-40, further comprising the step of separating the sorbate sorbed to the sorptive composite material from another sorbate sorbed to a sorptive composite material or from several sorbates sorbed to one or several sorptive composite materials.

42. Process according to claim 40 or 41, wherein the separation step is carried out by sieving, flotation, sedimentation, centrifugation, or magnetic interaction.

43. Process according to any of the claims 33-42, wherein further comprising the step of releasing the sorbate from the sorptive composite material.

44. Process according to claim 43, wherein the sorbate is a compound that is unstable or only temporarily formed under incubation, reaction, or cultivation conditions.

45. Process according to claim 44, wherein the sorbate is fed to the cell by culturing the cell in the presence of the sorbate sorbed to the sorptive composite material.

46. Process according to claims 44 or 45, wherein the cell is a virus or a prokaryotic or eukaryotic cell.

47. Process according to claim 34, wherein the sorptive composite material is incorporated in a chromatography column.
